## Europäisches Patentamt

## European Patent Office

## Office européen des brevets

(19)

(11) Veröffentlichungsnummer: **0 122 301 B1**

(12) **EUROPÄISCHE PATENTSCHRIFT**

(45) Veröffentlichungstag der Patentschrift:
28.01.87

(21) Anmeldenummer: **83103470.7**

(22) Anmeldetag: **09.04.83**

(51) Int. Cl.⁴: **A 61 M 16/01**

(54) **Narkosesystem.**

(43) Veröffentlichungstag der Anmeldung:
**24.10.84 Patentblatt 84/43**

(45) Bekanntmachung des Hinweises auf die Patenterteilung:
**28.01.87 Patentblatt 87/5**

(84) Benannte Vertragsstaaten:
**AT BE CH DE FR GB IT LI NL SE**

(56) Entgegenhaltungen:
**DE - A - 2 601 902**
**FR - A - 1 092 014**
**FR - A - 1 347 054**
**FR - A - 2 197 560**
**GB - A - 2 062 476**
**US - A - 3 721 238**

(73) Patentinhaber: **Drägerwerk Aktiengesellschaft, Moislinger Allee 53-55, D-2400 Lübeck 1 (DE)**

(72) Erfinder: **Torri, G., Prof., Viale Abruzzi, 70/A, I-20131 Milano (IT)**

## Beschreibung

Die Erfindung bezieht sich auf ein Narkoseatemsystem für Inhalationsnarkosen, welches zwischen halboffener und halbgeschlossener Betriebsart umschaltbar ist, mit einer einen $CO_2$-Absorber enthaltenden, einen Inspirationszweig und einen Exspirationszweig bildenden Ringleitung, wobei im Exspirationszweig ein durch Atemdruck gesteuertes Auslassventil angeordnet ist.

Narkosesysteme, welche zwischen halboffener und halbgeschlossener Betriebsart umrüstbar sind, gehören beispielsweise in der Ausführungsform «Narkosespiromat» durch den Prospekt 51/61 der Drägerwerke AG, 3. Ausgabe Juli 1970, zum Stande der Technik. Zur Umrüstung sind dabei verschiedene Handgriffe erforderlich.

Ein umschaltbares Narkosesystem ist in der GB-PS 1193522 beschrieben, ferner in der DE-OS 2945485. Dabei sind jeweils Umschaltventile vorgesehen, welche betriebsartabhängig den Kreislauf des Atemgases schliessen oder für den halboffenen Betrieb nach aussen öffnen.

Die vorbekannten umschaltbaren Ausführungen erscheinen dadurch nachteilig, dass die Umschaltung in die beiden Betriebsarten nicht genügend sinnfällig hervortritt und dass bei mehreren Bedienungseingriffen u. U. Fehlbedienungen möglich sind, die nicht sofort erkannt werden.

Die Erfindung geht von der Aufgabenstellung aus, bei einem Narkoseatemsystem der eingangs beschriebenen Art eine besonders einfache und sinnfällige Umschaltmöglichkeit zwischen halboffener und halbgeschlossener Betriebsart anzugeben und dadurch die Sicherheit des Systems zu erhöhen. Die Lösung dieser Aufgabenstellung erfolgt dadurch, dass der Absorber ein an ein Grundgehäuse des Narkoseatemsystems ansetzbares, optisch gut wahrnehmbares Zusatzgehäuse aufweist, dass der Absorber im Exspirationszweig angeordnet und als aus am Grundgehäuse festen Anschlussstellen lösbares Umschaltelement ausgebildet ist, und dass die eine feste Anschlussstelle des Absorbers ein durch das Umschaltelement zwangsweise betätigtes Absperrelement aufweist, welches beim Herausnehmen des Umschaltelements die Ringleitung unterbricht und getrennte Atemzweige herstellt, und dass die zweite beim Herausnehmen offene feste Anschlussstelle des Absorbers hinter dem steuerbaren Auslassventil liegt.

In dieser Ausführung wird das für die halbgeschlossene Betriebsart kennzeichnende Bestückungselement, nämlich der $CO_2$-Absorber, unmittelbar und sinnfällig als Umschaltelement verwendet. Bei angeschlossenem $CO_2$-Absorber liegt in jedem Falle halbgeschlossene Betriebsweise vor, während bei fehlendem, abgenommenen $CO_2$-Absorber mit Sicherheit davon ausgegangen werden kann, dass das System nunmehr in halboffener Betriebsweise arbeitet. Eine solche Anordnung ist bautechnisch einfach und schliesst die Gefahr von Fehlbedienungen aus.

Durch das Ansetzen eines deutlich wahrnehmbaren Beuteils, das in einem an das Grundgehäuse des Narkoseatemsystems ansetzbaren optisch gut wahrnehmbaren Zusatzgehäuse untergebracht ist, wird die Bedienungsperson auffällig über die von ihr gewählte Betriebsart informiert. Das Umschalten ist auch während der Durchführung der Anästhesie möglich, wodurch die Flexibilität des Systems erhöht wird.

Zweckmässig können zum Einsetzen und Lösen des Absorbers an den Anschlussstellen Schnellverbindungselemente vogesehen sein. Derartige Schnellverbindungselemente sind beispielsweise vorteilhaft als rastbare Steckverbindungen ausgebildet. Damit ergibt sich die Möglichkeit, den Absorber mit einem einzigen Handgriff anzuschliessen und zu lösen und gleichzeitig die sichere Umschaltung in die gewünschte Betriebsart vorzunehmen.

Ein weiterer Vorteil kann gegebenenfalls dadurch erreicht werden, dass der Absorber als durch eine Trennwand mit Durchlassspalt in zwei Kammern geteilter Kanister ausgebildet ist, und dass an beiden Anschlussstellen gleiche Anschlusselemente derart vorgesehen sind, dass das Ansetzen des Absorbers unter Vertauschung der Anschlussstellen möglich ist. Durch einen solchen symmetrischen Aufbau lässt sich die Atemkalkfüllung der beiden Kammern besser ausnutzen. Ist der Atemkalk in der ersten Kammer verbraucht, so kann der aus den gehäusefesten Anschlussstellen leicht lösbare Absorber um 180° gedreht und erneut angeschlossen werden, wodurch eine bevorzugte Ausnutzung des Atemkalks in der Teilkammer auf der Einlassseite erfolgt.

In der Zeichnung ist ein Ausführungsbeispiel des Gegenstandes der Erfindung schematisch dargestellt; es zeigen:

Fig. 1 ein Schaltbild für das Narkoseatemsystem gemäss der Erfindung,

Fig. 2 eine isometrische Ansicht eines Narkosegerätes zur Anwendung des angegebenen Narkoseatemsystems.

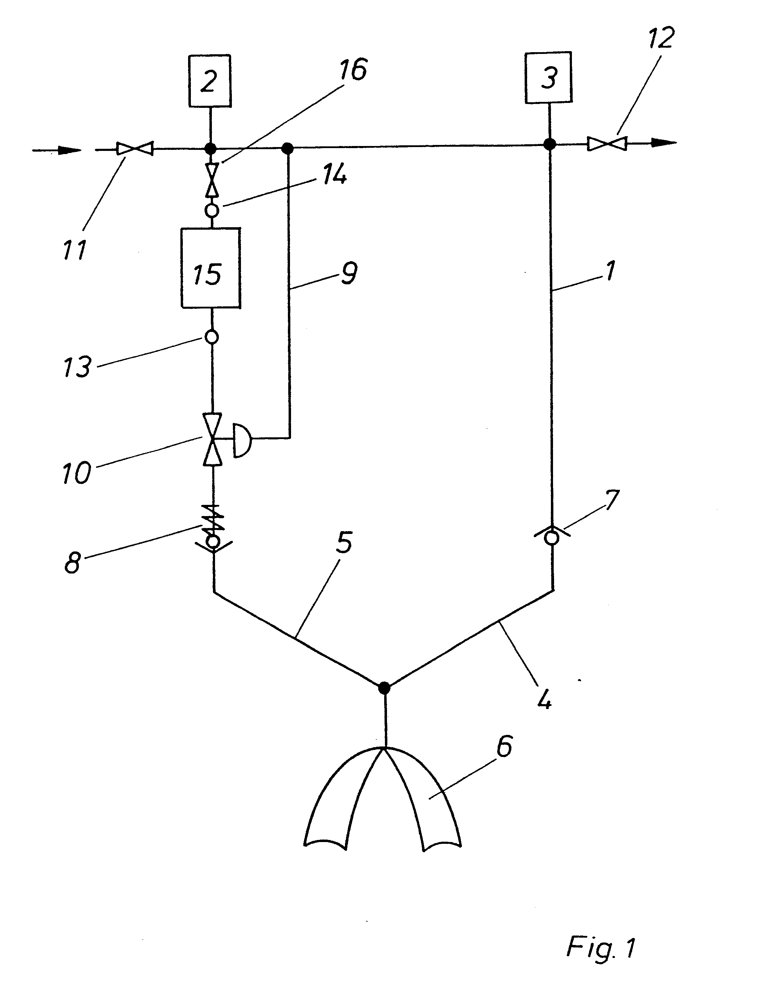

In Fig. 1 ist eine Ringleitung 1 dargestellt, in die von einer Frischgasquelle 2 oder durch ein Beatmungsgerät 3 (bzw. Handbeatmungsbeutel) Frischgas eingespeist wird. In den beiden Atemgaszweigen 4, 5 befinden sich vor dem Patientenausgang 6 Rückschlagventile 7, 8. Im Exspirationszweig 5 ist ausserdem ein über eine Verbindungsleitung 9 vom Beatmungsdruck steuerbares Auslassventil 10 angeordnet.

Aus Sicherheitsgründen sind ein Zusatzluftventil 11 und ein einstellbares Überdruckventil 12 vorgesehen. Das Zusatzluftventil 11 ermöglicht bei nicht ausreichender Frischgasversorgung das Einspeisen atmosphärischer Luft in das Beatmungssystem, und das Überdruckventil 12 ermöglicht einen Druckausgleich bei unzulässiger Druckerhöhung auf der Versorgungsseite. In Strömungsrichtung hinter dem Auslassventil 10 befindet sich ein an zwei Anschlussstellen 13, 14 eingeschalteter Absorber 15, der als lösbares Umschaltelement ausgebildet ist.

Bei halboffener Betriebsart wird der Inspirationszweig 4 aus der Frischgasquelle 2 oder durch das Beatmungsgerät 3 mit Frischgas versorgt.

Während der Inspiration durch Ausdrücken des Balges des Beatmungsgerätes oder des Handbeatmungsbeutels wird über das Rückschlagventil 7 Atemgas zum Patienten 6 gefördert. Dabei baut sich in der Steuerleitung 9 ein Druck auf, der das Auslassventil 10 schliesst. Der Patient wird mit Atemgas beatmet. Während der Exspiration durch Entlasten des Balges oder des Handbeatmungsbeutels sinkt der Druck in der Steuerleitung 9, und das Rückschlagventil 8 sowie das Auslassventil 10 werden nunmehr geöffnet. Damit entweicht das Exspirationsgas vom Patienten 6 über die offene bzw. mit einer Narkosefortleitung verbundene Anschlussstelle 13.

Der Absorber 15 ist bei dieser Betriebsart herausgenommen, und dadurch ist die Anschlussstelle 14 durch ein zwangsweise betätigtes Absperrelement 16 verschlossen, so dass anstelle einer geschlossenen Ringleitung 1 zwei getrennte Atemzweige 4, 5 vorliegen.

Zum Übergang in die halbgeschlossene Betriebsart wird das bestimmende Bauelement, nämlich der Absorber 15, zwischen die Anschlussstellen 13, 14 eingesetzt. Dabei öffnet das Absperrelement 16 an der Anschlussstelle 14 zwangsweise und schliesst die beiden Atemzweige 4, 5 zu einer geschlossenen Ringleitung 1 zusammen, in die von der Frischgasquelle 2 Frischgas eingespeist werden kann.

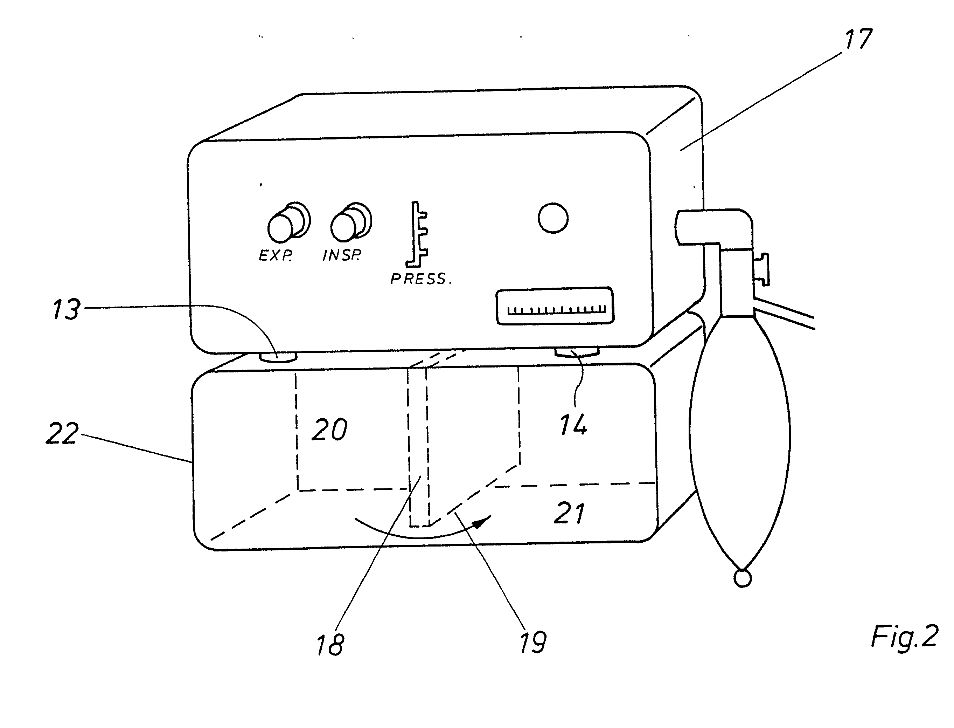

In Fig. 2 ist eine gerätetechnische Ausführungsform dargestellt, in der das Narkoseatemsystem in einem Grundgehäuse 17 untergebracht ist. Der Absorber 15 ist hierbei als Kanister 22 mit gleichen Breiten- und Tiefenmassen wie das Grundgehäuse 17 des Narkoseatemsystems ausgebildet. Dadurch wird eine besonders sinnfällige und optisch gut wahrnehmbare Anzeige der vorliegenden Betriebsart erreicht.

An den Anschlussstellen 13, 14 befinden sich einklinkbare Schnellverbindungselemente, welche einen gasdichten Anschluss ermöglichen. An der Anschlussstelle 14 ist als zwangsweise betätigtes Absperrelement 16 ein Rückschlagventil vorgesehen. Die Anschlussstelle 13 enthält ein Schnellverbindungselement, welches mit einem angepassten Anschlusselement einer Narkosegasfortleitung verbunden werden kann.

Wie der gestrichelt angedeutete Innenaufbau des Kanisters 22 erkennen lässt, sind zwei durch eine Trennwand 18 getrennte Kammern 20, 21 vorgesehen und über einen Durchlassspalt 19 am Kanisterboden miteinander verbunden. Beide Kammern 20, 21 weisen eine Füllung von Absorbermaterial auf, welches auch den Durchlassspalt 19 ausfüllt. Durch Drehen des Kanisters 22 um 180° lässt sich die zunächst auf der Auslassseite liegende Kammer 21 nunmehr an der Einlassseite anschliessen, so dass eine bessere Ausnutzung der Absorberfüllung erreicht wird.

## Patentansprüche

1. Narkosesystem für Inhalationsnarkosen, welches zwischen halboffener und halbgeschlossener Betriebsart umschaltbar ist, mit einer einen $CO_2$-Absober (15) enthaltenden, einen Inspirationszweig (4) und einen Exspirationszweig (5) bildenden Ringleitung, wobei im Exspirationszweig ein durch Atmungsdruck gesteuertes Auslassventil (10) angeordnet ist, dadurch gekennzeichnet, dass der Absorber (15) ein an ein Grundgehäuse (17) des Narkoseatemsystems ansetzbares, optisch gut wahrnehmbares Zusatzgehäuse aufweist, dass der Absorber (15) im Exspirationszweig (5) angeordnet und als aus am Grundgehäuse festen Anschlussstellen (13, 14) lösbares Umschaltelement ausgebildet ist und dass die eine feste Anschlussstelle (14) ein durch das Umschaltelement (15) zwangsweise betätigtes Absperrelement (16) aufweist, welches beim Herausnehmen des Umschaltelements (15) die Ringleitung (1) unterbricht und getrennte Atemzweige (4, 5) herstellt, und dass die zweite beim Herausnehmen offene feste Anschlussstelle (13) hinter dem steuerbaren Auslassventil (10) liegt.

2. Narkoseatemsystem nach Anspruch 1, dadurch gekennzeichnet, dass zum Einsetzten und Lösen des Absorbers (15) an den Anschlussstellen (13, 14) Schnellverbindungselemente vorgesehen sind.

3. Narkoseatemsystem nach Anspruch 2, dadurch gekennzeichnet, dass die Schnellverbindungselemente rastbare Steckverbindungen (13, 14) sind.

4. Narkoseatemsystem nach Anspruch 1, dadurch gekennzeichnet, dass der Absorber (15) als durch eine Trennwand (18) mit Durchlassspalt (19) in zwei Kammern (20, 21) geteilter Kanister (22) ausgebildet ist und dass an beiden Anschlussstellen (13, 14) gleiche Anschlusselemente derart vorgesehen sind, dass das Ansetzen des Absorbers (15) unter Vertauschung der Anschlussstellen (13, 14) möglich ist.

## Claims

1. An anaesthesia system for anaesthetics to be inhaled, which system can be switched over between half-open and half-closed operating modes, with a closed circuit, containing a $CO_2$-absorber (15) and comprising an inspiratory branch (4) and an expiratory branch (5), an outlet valve (10) controlled by breathing pressure being arranged in the expiratory branch, characterised in that: the absorber (15) has a visually well discernable additional housing, which can be connected to a base housing (17) of the anaesthetic respiratory system; in that the absorber (15) is arranged in the expiratory branch (5) and comprises a switch-over element detachable from fixed connection points (13, 14) on the base housing; in that one fixed connection point (14) has a blocking element (16), which is forced to be operated by the switch-over element (15), and which, on the removal of the switch-over element (15), interrupts the closed circuit (1) and produces separate respiratory branches (4, 5); and in that the second fixed connection point (13), open on said removal, lies behind the controllable outlet valve (10).

2. An anaesthetic respiratory system according to claim 1, characterised in that, for inserting and releasing the absorber (15), quick release elements are provided at the connection points (13, 14).

3. An anaesthetic respiratory system according to claim 2, characterised in that the quick release elements are lockable plug-in connections (13, 14).

4. An anaesthetic respiratory system according to claim 1, characterised in that the absorber (15) is formed as a cannister (22) which is divided into two chambers (20, 21) by a dividing wall (18) with an outlet gap (19); and in that similar connection elements are provided at both connection points (13, 14), such that the replacement of the absorber (15) is possible by exchanging the connection points (13, 14).

**Revendications**

1. Système d'anesthésie pour anesthésies par inhalation, qui peut êre commuté entre un mode de fonctionnement semi-ouvert et un mode de fonctionnement semi-fermé, avec une conduite circulaire contenant un absorbeur (15) de $CO_2$ et formant une branche d'inspiration (4) et une branche d'expiration (5), une soupape de sortie (10) asservie par la pression respiratoire étant disposée dans la branche d'expiration (5), caractérisé en ce que l'absorbeur (15) présente un bâti auxiliaire bien reconnaissable visuellement qui peut être monté sur le bâti de base (17) du système respiratuer d'anesthésie, en ce que l'absorbeur (15) est disposé dans la branche d'expiration (5) et est réalisé sous la forme d'un élément de commutation monté de manière amovible en des points de raccordement fixes (13, 14) du bâti de base, en ce que l'un (14) des points de raccordement fixes présente un élément d'arrêt (16) qui est actionné de manière forcée par l'élément de commutation (15) et qui interrompt la conduite circulaire (1) lors de la dépose de l'élément de commutation (15) et réalise des branches respiratoires séparées (4, 5), et en ce que le deuxième point de raccordement fixe (13), qui est ouvert lors de la dépose, se trouve derrière la soupape de sortie asservissable (10).

2. Système d'anesthésie selon la revendication 1, caractérisé en ce qu'il est prévu aux points de raccordement (13, 14) des éléments d'assemblage rapide pour mettre en place et déposer l'absorbeur (15).

3. Système d'anesthésie selon la revendication 2, caractérisé en ce que les éléments d'assemblage rapide sont des connecteurs encliquetables (13, 14).

4. Système d'anesthésie selon la revendication 1, caractérisé en ce que l'absorbeur (15) est réalisé sous la forme d'une boîte-bidon (22) divisé en deux compartiments (20, 21) par une paroi séparatrice (18) munie d'une fente de passage (19), et en ce qu'il est prévu des éléments de raccordement identiques aux deux points de raccordement (13, 14), de sorte qu'il est possible de monter l'absorbeur (15) en inversant les points de raccordement (13, 14).

*Fig. 1*

EXP.  INSP.  PRESS.

17

13

22

20

14

21

18    19

Fig.2

0122301